Europäisches Patentamt
European Patent Office
Office européen des brevets

(11) Numéro de publication: **0 002 400**
**A1**

# (12) DEMANDE DE BREVET EUROPEEN

(21) Numéro de dépôt: 78400172.9

(22) Date de dépôt: 10.11.78

(51) Int. Cl.²: **C 07 D 513/04**
A 61 K 31/425, A 61 K 31/505
//(C07D513/04, 277/00, 235/00),
(C07D513/04, 277/00, 239/00)

(30) Priorité: **24.11.77 FR 7735305**
**16.10.78 FR 7829414**

(43) Date de publication de la demande:
13.06.79 Bulletin 79/12

(84) Etats contractants désignés:
BE CH DE FR GB LU NL SE

(71) Demandeur: **SYNTHELABO**
**1, avenue de Villars**
**F-75341 Paris Cedex 07(FR)**

(72) Inventeur: **Bigg, Dennis Claude**
**24, rue Curie**
**F-34590 Marsillargues(FR)**

(74) Mandataire: **Thouret-Lemaitre, Elisabeth**
**Service Brevets - SYNTHELABO 58, rue de la Glacière**
**F-75621 Paris Cedex 13(FR)**

(54) **Dérivés de thiazole, leur préparation, intermédiaires et leur application en thérapeutique.**

(57) Dérivés de thiazole répondant à la formule (I)

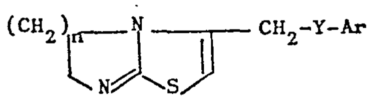

dans laquelle
n est 1 ou 2,
Y représente O, S ou $SO_2$,
et Ar est un radical phényle, naphtyle ou phényle portant un ou plusieurs substituants choisis dans le groupe constitué par les atomes d'halogène, $NO_2$, $CF_3$, les radicaux phényle, alkyles, alcoxy, alkylthio, hydroxy-alkyles, alcényles, acyles, benzoyle et benzyloxy, doués d'une activité psychotropique.
On prépare ces dérivés par réaction entre un composé de formule:

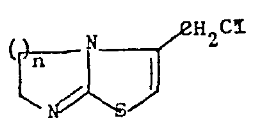

et un composé ArYH.

EP 0 002 400 A1

TITRE MODIFIÉ
voir page de garde



Dérivés de thiazole , leur préparation et leur application en thérapeutique.

La présente invention concerne des dérivés de thiazole, leurs sels d'addition aux acides pharmaceutiquement acceptables, leur préparation et leur application en thérapeutique.

Les composés de l'invention répondent à la formule (I)

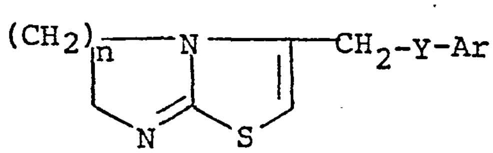

(I)

dans laquelle
n est 1 ou 2,
Y représente O, S ou $SO_2$,
et Ar est un radical phényle, naphtyle ou phényle portant un ou plusieurs substituants choisis dans le groupe constitué par les atomes d'halogène, $NO_2$, $CF_3$, les radicaux phényle, alkyles, alcoxy, alkylthio, hydroxy-alkyles,

alcényles, acyles, benzoyle et benzyloxy, les radicaux acyles et alkyles droits ou ramifiés ayant de 1 à 4 atomes de carbone,
les radicaux alcényles ayant de 2 à 4 atomes de carbone.

Les composés préférés de l'invention sont ceux pour lesquels n est 1 et le radical Ar est un phényle ou un phényle portant un substituant en position ortho.

Selon l'invention on prépare les composés de l'invention par réaction entre un composé

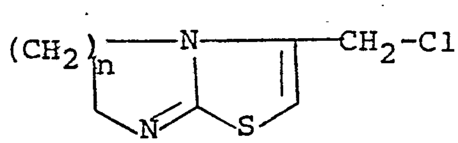

(II)

et un composé ArYH (III)
n et Y ayant les définitions données ci-dessus.

Le composé de formule (II) dans lequel n est 1 est nouveau et est obtenu par réaction entre l'imidazolinethione -2 et la dichloro-1,3 acétone selon le schéma réactionnel.

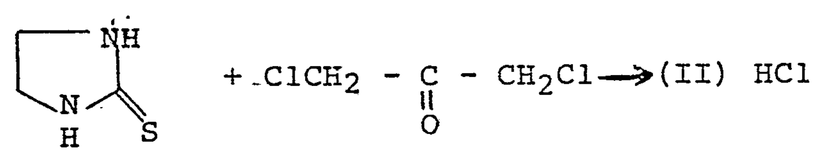

Les composés pour lesquels Y est $SO_2$ peuvent être obtenus par oxydation du composé correspondant dans lequel Y est S. L'oxydation peut être effectuée à l'aide d'eau oxygénée.
La condensation entre les composés (II) et (III) est effectuée en présence d'une base telle que NaOH et dans un solvant approprié. Des solvants aprotiques dipolaires tels que le diméthylsulfoxyde (DMSO) sont particulièrement avantageux.

La température est la température ambiante ou une température plus élevée.
Le composé (III) peut être utilisé en excès de 1 à 10 par rapport au composé (II).

Les composés de l'invention sont des psychotropes.

Les exemples ci-après illustrent l'invention. Les analyses et spectres IR et RMN confirment la structure des composés.

Préparation des composés de départ

1. Chlorhydrate du chlorométhyl-3 dihydro-5,6 imidazo [2,1-b] thiazole (II)

On ajoute en une fois 102 g (1 mole) d'imidazolidine-2-thione à une solution de 127 g (1 mole) de dichloro-1,3 acétone dans 200 ml de n-butanol, puis on chauffe la suspension à la température de reflux. On obtient une solution claire. Un précipité blanc se forme au bout de 30 mn environ. Après 4 heures de reflux on laisse refroidir le mélange réactionnel, on filtre le précipité puis le lave avec successivement de l'éthanol, de la diméthylcétone et de l'oxyde d'éthyle.
Les cristaux blancs obtenus fondent à 229-231°C en se décomposant.

2. Chlorhydrate de chlorométhyl-3 dihydro-6,7 [5H] - thiazolo [3,2a] pyrimidine.

On chauffe à reflux pendant 2 heures une solution de dichloro-1,3 acétone (12,7g - 0,1 mole) et de tétrahydro-1,4,5,6 pyrimidine-thiol (11,6 g - 0,1 mole) dans du n-butanol (50ml). On filtre le précipité beige qui se forme lors du refroidissement et on le recristallise dans de l'éthanol.
Le composé obtenu fond à 219 - 221°C avec décomposition.

EXEMPLE 1 : Chlorhydrate du phénoxyméthyl-3 dihydro-5,6 imidazo [2,1-b] thiazole. [Y = O - n = 1 - Ar = $C_6H_5$]

Tout en refroidissant et agitant, on ajoute, par portions, en 10 mn, 42,2 g (0,2 mole) du composé (II) obtenu précédemment à une solution refroidie de phénoxyde de sodium [solution préparée à partir de 37,6 g (0,4 mole) de phénol et de 24 g (0,6 mole) de NaOH] dans un mélange de 250 ml de DMSO et 50 ml d'eau.
On agite le mélange réactionnel pendant la nuit à la température ambiante, on le verse dans de l'eau (500 ml) et on l'extrait 4 fois avec 250 ml d'acétate d'éthyle. On lave les extraits avec 1 l d'une solution $\frac{N}{10}$ de $NH_4OH$ et 4 fois avec 1 l d'eau puis on les sèche sur $MgSO_4$. Après évaporation on obtient une huile brune qui se solidifie. On reprend le solide dans un mélange éthanol/chloroforme et on le traite avec un mélange éther/acide chlorhydrique. On filtre le précipité formé, le lave avec de l'acétone et de l'éther et on le fait recristalliser dans un mélange éthanol/heptane. On obtient le composé sous forme de cristaux blancs fondant à 203-4°C.

EXEMPLE 2 : Chlorhydrate de phénylthiométhyl-3 dihydro-5,6 imidazo [2,1 - b] thiazole. [Y = S n = 1 Ar = $C_6H_5$]

On ajoute une solution de chlorhydrate de chlorométhyl-3 dihydro- 5,6 imidazo [2,1 - b] thiazole (10,55 g - 0,05 mole) dans un mélange 50/50 de DMSO et eau à une solution refroidie de thiophénol (5,5g - 0,05 mole), DMSO (75 ml), eau (15 ml) et NaOH (4 g - 0,1 mole). On agite le mélange réactionnel toute la nuit sous atmosphère d'azote, on le verse dans de l'eau et on extrait avec de l'acétate d'éthyle. On lave les extraits avec de l'eau, on sèche sur $MgSO_4$, on filtre et évapore. On reprend l'huile orange dans de l'éthanol et on la traite avec un mélange $Et_2O/HCl$.

On obtient le chlorhydrate recherché que l'on recristallise dans de l'alcool isopropylique. On obtient des cristaux blancs. F = 196-7°C.

EXEMPLE 3 : Chlorhydrate de (méthyl-2 phénoxy-méthyl)-3 dihydro-5,6 imidazo- [2,1-b] thiazole.
[Y = O n = 1 Ar = 2-$CH_3$-$C_6H_4$]

On ajoute du chlorhydrate de chlorométhyl-3 dihydro-5,6 imidazo [2,1-b] -thiazole (5,28 g - 0,025 mole) par portions, à une solution d'o-crésol (5,4g - 0,05 mole) dans un mélange 75/15 de DMSO/eau contenant de la soude (3,0g - 0,075 mole) pendant 5-10mn, tout en refroidissant. On agite le mélange réactionnel à la température ambiante pendant la nuit, on le rend basique à l'aide de $NH_4OH$ et on extrait avec EtOAc. On lave les extraits avec $NH_4OH$ et de l'eau puis on sèche sur $MgSO_4$ et évapore. On reprend l'huile obtenue dans de l'éthanol et on traite avec $Et_2O$/HCl. On recristallise les cristaux blancs obtenus dans un mélange EtOH/EtOAc.

F = 215 - 7°C (décomposition).

EXEMPLE 4 : Chlorhydrate de (benzoyl-2 phénoxyméthyl) -3 dihydro-5,6 imidazo [2,1-b] thiazole.
[Y = O n = 1 Ar = 2-$C_6H_5$CO-$C_6H_4$]

On ajoute, goutte à goutte, en 40 mn, une solution de chlorhydrate de (chlorométhyl-3 dihydro-5,6 imidazo- [2,1-b] thiazole (5,28g - 0,025 mole) dans un mélange 50/10 de DMSO/eau à une solution de NaOH (3,0g-0,075 mole) et d'ortho-hydroxy-benzophénone (9,98 g - 0,05 mole) dans un mélange 25/15 de DMSO/eau à 75-80°C. On maintient cette température pendant une demi-heure après la fin de l'addition du composé chloré. On dilue le mélange réactionnel avec de l'eau et on extrait avec de l'acétate d'éthyle. On lave les extraits avec de l'ammoniaque diluée et de

l'eau puis on sèche sur $MgSO_4$. On évapore et on traite avec un mélange éther/HCl. On obtient un solide jaune que l'on recristallise. F = 205-6°C.

EXEMPLE 5 : Phénylsulfonylméthyl-3 dihydro-5,6 imidazo [2,1-b] thiazole. [Y = $SO_2$ n = 1 Ar = $C_6H_5$]

On ajoute du peroxyde d'hydrogène (18,4 ml d'eau oxygénée à 110 volumes - 0,09 mole) à une solution du composé de l'exemple 1 (4,5g - 0,018 mole) dans de l'acide acétique (50 ml) tout en agitant et refroidissant au bain de glace. On agite la solution limpide pendant 16 heures à la température ambiante puis on la porte au reflux pendant 4 heures; on laisse refroidir et on concentre sous pression réduite. On dilue la solution avec de l'eau, on alcalinise avec KOH et on extrait avec $CHCl_3$. On évapore les extraits séchés sur $MgSO_4$. On obtient un solide blanc que l'on recristallise dans de l'acétone. F = 168 - 169,5°C.

EXEMPLE 6 : Chlorhydrate de phénoxyméthyl-3 dihydro-6,7 [5H] thiazolo [3,2 a] pyrimidine. [Y = O n = 2 Ar = $C_6H_5$]

On ajoute, par portions, du chlorhydrate de chlorométhyl-3 dihydro-6,7 [5H] thiazolo [3,2-a] pyrimidine (4,5g - 0,02 mole) à un mélange de phénol (3,76 g - 0,04 mole), NaOH (1,76g - 0,044 mole), DMSO (60 ml) et eau (12 ml), tout en agitant. Après 24 h à la température ambiante, on dilue le mélange réactionnel avec de l'eau, on alcalinise et on extrait avec ETOAc. On lave les extraits avec de la potasse aqueuse et de l'eau.
On sèche sur $MgSO_4$ et on évapore. On obtient une huile jaune que l'on dissout dans de l'éthanol et traite avec un mélange éther/HCl. On obtient un solide jaune que l'on recristallise dans un mélange alcool isopropylique/éther de pétrole. F = 203-5°C.

Dans le tableau ci-après sont représentés les composés de l'invention préparés à titre d'exemples.

TABLEAU I

| Composé n° | Y | Ar | Sel ou base | F°C | n |
|---|---|---|---|---|---|
| 1(ex.2) | S | $C_6H_5$ | HCl | 196-7 | 1 |
| 2 | S | $4\text{-}ClC_6H_4$ | base | 77,8-78,5 | 1 |
| 3 | O | $2\text{-}MeOC_6H_4$ | hemi-oxalate 1 $H_2O$ | 171-2,5 | 1 |
| 4 | O | $4\text{-}ClC_6H_4$ | HCl | 216-8 | 1 |
| 5(ex.1) | O | $C_6H_5$ | HCl | 203-4 | 1 |
| 6 | O | $4\text{-}MeOC_6H_4$ | HCl | 198-9 | 1 |
| 7(ex.5) | $SO_2$ | $C_6H_5$ | base | 168-9,5 | 1 |
| 8 | O | $3\text{-}MeOC_6H_4$ | HCl* | 205-6,5 | 1 |
| 9 | O | $3\text{-}ClC_6H_4$ | HCl | 229-31 | 1 |
| 10(ex.3) | O | $2\text{-}MeC_6H_4$ | HCl | 215-7 | 1 |
| 11 | O | $2\text{-}ClC_6H_4$ | HCl | 227-8,5 | 1 |
| 12 | O | $4\text{-}MeC_6H_4$ | HCl | 223,5-224,5 | 1 |
| 13 | O | $3\text{-}MeC_6H_4$ | HCl* | 206-7,5 | 1 |

| Composé n° | Y | Ar | Sel ou base | F°C | n |
|---|---|---|---|---|---|
| 14 | O | 2,3-$Cl_2C_6H_3$ | HCl | 234-5 | 1 |
| 15 | O | 2-allyl$C_6H_4$ | HCl | 206,5-207,5 | 1 |
| 16 | O | 2-$CH_3CO$-$C_6H_4$ | HCl* | décomposition | 1 |
| 17 | O | 2-$HOCH_2C_6H_4$ | HCl | 195-6,5 | 1 |
| 18 | O | 2-$BrC_6H_4$ | HCl | 207,5-208,5 | 1 |
| 19 | S | 2-$MeOC_6H_4$ | HCl | 217-9 | 1 |
| 20 | O | 2-$FC_6H_4$ | HCl | 229,5-230,5 | 1 |
| 21 | O | 2-$CF_3C_6H_4$ | HCl | 202-3 | 1 |
| 22 | O | 2-i$PrC_6H_4$ | HCl | 214-5 | 1 |
| 23 | O | 2-$MeSC_6H_4$ | HCl | 215,5-216,5 | 1 |
| 24 | O | 2-$EtOC_6H_4$ | HCl | 184,5-185,5 | 1 |
| 25 | O | 2,5-$Cl_2C_6H_3$ | HCl | 234-6 | 1 |
| 26 | O | 2,4-$Cl_2C_6H_3$ | HCl | 239-40 | 1 |
| 27 (ex.4) | O | 2-$PhCOC_6H_4$ | HCl | 205-6 | 1 |
| 28 | S | $C_6H_5$ | Hemi- * oxalate | 198-9 | 2 |
| 29 (ex.6) | O | $C_6H_5$ | HCl | 203-5 | 2 |

* Sel partiellement hydraté

| Composé n° | Y | Ar | Sel ou base | F(°C) | n |
|---|---|---|---|---|---|
| 30 | O | 1-naphtyl | HCl | 194-6 | 1 |
| 31 | O | 2-$C_6H_5$-$C_6H_4$ | HCl | 234-6 | 1 |
| 32 | O | 2-Cl-4-$NO_2$-$C_6H_3$ | HCl | 206-7 | 1 |
| 33 | O | 2,6-$Cl_2$-$C_6H_3$ | HCl* | 206-8 | 1 |
| 34 | O | 4 - F - $C_6H_4$ | HCl | 218-9 | 1 |
| 35 | O | 3,4-di Cl-$C_6H_3$ | HCl | 248,5-250 | 1 |
| 36 | O | 3-$CF_3$-$C_6H_4$ | HCl | 216-7 | 1 |
| 37 | O | 2,6-di$CH_3$-$C_6H_3$ | HCl | 272,5-274 | 1 |
| 38 | O | 4-$C_6H_5$ - $C_6H_4$ | HCl | 244,5-246 | 1 |
| 39 | O | 2-$C_6H_5CH_2O$-$C_6H_4$ | HCl | 222-4 | 1 |

Les composés de l'invention ont été soumis à des essais pharmacologiques qui ont mis en évidence leurs propriétés psychotropes.

La toxicité des composés a été déterminée sur la souris, par voie intrapéritonéale. La DL 50 va d'environ 10 à environ 100 mg/kg.

Par ailleurs on a étudié l'action des drogues sur les pointes ponto-géniculo-occipitales lors du syndrome réserpinique chez le chat curarisé, afin de mettre en évidence les propriétés antidépressives des composés.

Chez le chat, on enregistre une activité spécifique au niveau du pont, du noyau genouillé latéral et du cortex occipital qui fut appelée pointes ponto-géniculo-occipitales (P.G.O.).

Cette activité spontanée, (pointes P.G.O.), apparaissant lors du cycle veille-sommeil, peut être induite par la réserpine, agent pharmacologique diminuant le taux des monoamines cérébrales.

Cette activité électroencéphalographique, modulée par des mécanismes neuronaux sous le contrôle de neurotransmetteurs synaptiques, constitue donc un test pharmacologique pour l'étude de l'action centrale des drogues.

Toutes les expériences sont réalisées de manière aiguë sur des chats des deux sexes de 2 à 3 kg.

Avant le début de la préparation chirurgicale effectuée sous narcose à l'éther, l'animal reçoit une injection intrapéritonéale de 0,75 mg/kg i.p. de réserpine.

Le chat est intubé pour permettre une ventilation artificielle.

Un anesthésique local (xylocaïne à 2%) est injecté au niveau des points de pression et des incisions.

Des canules sont placées au niveau de la veine fémorale, pour l'injection des produits et l'infusion d'un agent curarisant, la gallamine triethiodide (Flaxédil$^{R}$), et de l'artère fémorale pour l'enregistrement de la pression artérielle.

Des électrodes monopolaires sont vissées au niveau cortical et des électrodes bipolaires coaxiales sont placées stéréotaxiquement au niveau des noyaux genouillés latéraux.

L'animal curarisé et ventilé artificiellement est maintenu à température constante tout au long de l'expérience.

L'électroencéphalogramme, l'électrocardiogramme et la pression artérielle sont enregistrés à l'aide d'un polygraphe : Grass modèle 79 D.

Des doses croissantes (0,1; 0,3; 1; 3; 10 et 30 mg/kg) des produits à étudier sont injectées par voie intraveineuse toutes les 30 minutes, 4H30 après l'administration de la réserpine.

Le nombre des pointes P.G.O. est comptabilisé automatiquement par périodes de 10 minutes et exprimé en pourcentage par périodes de 30 minutes en prenant la valeur obtenue lors des 30 minutes de contrôle comme référence (100%).

La dose efficace réduisant de 50% (DE 50) le nombre des pointes P.G.O. est calculée à l'aide d'une courbe de regression semi-logarithmique.

Les résultats sont rapportés dans le tableau ci-joint. (II)

TABLEAU II

| Composé n° | P G O DE 50 (mg/kg i.v. chat) |
|---|---|
| [illegible] | 0,4 |
| [illegible] | 0,4 |
| [illegible] | 1,2 |
| [illegible] | 1 |
| [illegible] | 1 |
| 1[illegible] | 0,3 |
| 1[illegible] | 0,24 |
| 1[illegible] | 0,8 |
| 1[illegible] | 0,14 |
| 2[illegible] | 0,2 |
| 2[illegible] | 0,5 |
| 3[illegible] | 0,24 |

Les résultats des essais précédents montrent que les composés de l'invention agissent sur le système nerveux central et sont utiles pour le traitement des troubles de l'humeur et de leurs symptomes associés.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc. avec tout excipient approprié.

La posologie quotidienne peut aller de 10 à 100 mg.

Revendications de brevet

1. Dérivés de thiazole répondant à la formule (I)

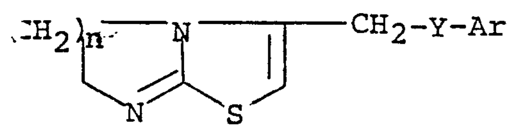

dans laquelle
n est 1 ou 2,
Y représente O, S ou $SO_2$,
et Ar est un radical phényle, naphtyle ou phényle portant un ou plusieurs substituants choisis dans le groupe constitué par les atomes d'halogène, $NO_2$, $CF_3$, les radicaux phényle, alkyles, alcoxy, alkylthio, hydroxy-alkyles, alcényles, acyles, benzoyle et benzyloxy, les radicaux acyles et alkyles droits ou ramifiés ayant de 1 à 4 atomes de carbone,
les radicaux alcényles ayant de 2 à 4 atomes de carbone,
ainsi que leurs sels d'addition aux acides pharmaceutiquement acceptables.

2. Dérivés selon la revendication 1, dans lesquels n est 1.

3. Dérivés selon la revendication 2, dans lesquels Ar est un phényle ou un phényle portant en ortho un des substituants spécifiés dans la revendication 1.

4. Dérivés selon la revendication 3, dans lesquels Y est O.

5. Le phénoxyméthyl-3 dihydro-5,6 imidazo [2,1-b]thiazole et son chlorhydrate.

6. Le (phényl-2 phénoxy)-méthyl-3 dihydro-5,6 imidazo [2,1-b] thiazole et son chlorhydrate.

7. Procédé de préparation des composés selon la revendication 1, procédé caractérisé en ce qu'on fait réagir un composé (II

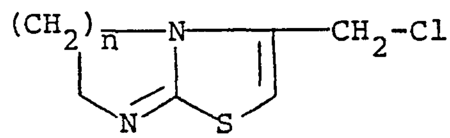

avec un composé ArYH (III),
Ar et Y ayant les significations données dans la revendication 1.

8. Médicament caractérisé en ce qu'il contient un des composés spécifiés dans l'une quelconque des revendications 1 à 6.

9. Dérivé de thiazole utile pour la préparation des composés de la revendication 1 et répondant à la formule

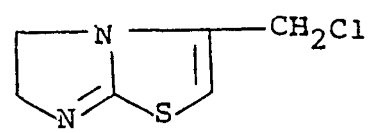

10. Procédé de préparation du composé selon la revendication 9, procédé caractérisé en ce qu'on fait réagir l'imidazolinethione de formule

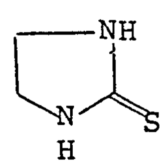

avec le composé $ClCH_2-CO-CH_2Cl$.

0002400

Numéro de la demande

Office européen des brevets

# RAPPORT DE RECHERCHE EUROPEENNE

EP 78 40 0172

| DOCUMENTS CONSIDERES COMME PERTINENTS | | | CLASSEMENT DE LA DEMANDE (Int. Cl.²) |
|---|---|---|---|
| Catégorie | Citation du document avec indication, en cas de besoin, des parties pertinentes | Revendication concernée | |
| | FR - A - 2 260 344 (PLANTEX)<br>* Revendications 1 et 11 *<br>-- | 1,8 | C 07 D 513/04<br>A 61 K 31/425<br>31/505//<br>(C 07 D 513/04<br>277/00<br>235/00)<br>(C 07 D 513/04<br>277/00<br>239/00) |
| A | FR - A - 2 223 371 (ICI)<br>* Revendications 1 et 5 *<br>---- | 1,8 | |
| | | | **DOMAINES TECHNIQUES RECHERCHES (Int. Cl.²)** |
| | | | C 07 D 513/04<br>A 61 K 31/425<br>31/505//<br>(C 07 D 513/04<br>277/00<br>235/00)<br>(C 07 D 513/04<br>277/00<br>235/00) |
| | | | **CATEGORIE DES DOCUMENTS CITES**<br>X: particulièrement pertinent<br>A: arrière-plan technologique<br>O: divulgation non-ecrite<br>P: document intercalaire<br>T: théorie ou principe a la base de l'invention<br>E: demande faisant interférence<br>D. document cite dans la demande<br>L: document cité pour d'autres raisons<br>&: membre de la même famille, document correspondant |
| X | Le présent rapport de recherche a été établi pour toutes les revendications | | |

| Lieu de la recherche | Date d'achèvement de la recherche | Examinateur |
|---|---|---|
| La Haye | 16-02-1979 | ALFARO |